# EUROPÄISCHE PATENTANMELDUNG

(11) **EP 2 005 973 A1**
(43) Veröffentlichungstag der Anmeldung: **24.12.2008**
(21) Anmeldenummer: 07110908.6
(22) Anmeldetag: 22.06.2007
(51) Int. Cl.: A61K 51/12

(54) **Positronen emittierende anorganische Partikel enthaltende Zusammensetzungen und deren Verwendung in der Medizin, insbesondere für diagnostische Verfahren**

(71) Anmelder: nanoPET Pharma GmbH, 10115 Berlin (DE)
(72) Erfinder: Schilling, Kristian, 10627 Berlin (DE)
(74) Vertreter: Vossius & Partner

(57) **Zusammenfassung**

Die Erfindung betrifft pharmazeutische Mittel, enthaltend eine partikuläre anorganische Matrix mit einem Durchmesser von 0,1nm bis 100µm, vorzugsweise 1nm bis 10µm, besonders bevorzugt 1nm bis 1µm wie beispielsweise Topas, (Al₂F₂)[SiO₄] und Chiolith, Na[Al₃F₄], bevorzugt Wavellit, Al₃(PO₄)₂(OH, F)₂, Calciumcarbonat, CaCO₃, Maghemit, γ-Fe₂O₃ ,besonders bevorzugt Zeolithe, allg. Formel Mₙ[(AlO₂)ₓ(SiO₂)_{y}] (M= Metall z.B. Na), Magnetit, Fe₃O₄, und Bariumsulfat, BaSO₄, sowie ganz besonders bevorzugt Galliumphosphat, GaPO₄, Apatit bzw. Fluorhydroxylapatit, Ca₅(PO₄)₃(OH, F) = 3Ca₃(PO₄)₂* Ca(OH, F)₂, und Flußspat, CaF₂, die neben der natürlichen Isotopenverteilung der Strukturtypen bildenden Elemente der Anionen bzw. Kationen auch medizinisch nutzbare Anteile an Positronen emittierenden Nukliden wie beispielsweise [15]O, [30]P, [13]N, bevorzugt [65]Ga,[11]C, besonders bevorzugt [131]Ba, [26]Al, sowie ganz besonders bevorzugt [68]Ga bzw. [18]F enthalten, deren Herstellung, sowie die Verwendung dieser Zusammensetzungen in der Medizin, besonders bevorzugt in der diagnostischen Bildgebung, insbesondere der Positronen-Emissions-Tomographie (PET), an Tier und Mensch sowie der in-vitro Diagnostik.

## Beschreibung

### Stand der Technik

### Positronen-Emissions-Tomographie (PET)-Nuklide

Von den mehr als 50 bekannten Positronenstrahlern sind nur einige wenige medizinisch einsetzbar. Die heute wichtigsten PET-Nuklide sind [18]F, [11]C, [13]N und [15]O. Sie zeichnen sich durch ihre extrem kurze Halbwertszeit aus, die von ca. 110 min bei [18]F bis zu etwa 2 min bei [15]O reicht.

Diese kurzen Halbwertszeiten stellen zwar enorme Ansprüche an die Bereitstellung und messtechnische Auswertung, bringen allerdings für den Patienten den Vorteil der geringen Strahlenbelastung aufgrund einer sehr kurzen Expositionsdauer.

Ein weiterer Vorteil dieser PET-Nuklide besteht darin, dass prinzipiell jedes organische Molekül mit ihnen radioaktiv markiert werden kann. Jedes organische Molekül besitzt ein oder mehrere Kohlenstoffatome (in der Regel: [12]C), von denen eines durch ein [11]C ersetzt werden kann; viele organische Moleküle tragen außerdem ein Sauerstoff- oder Stickstoffatom.

Fluor kommt in natürlichen und synthetischen Verbindungen zwar relativ selten vor, doch lässt es sich durch Substitution von Wasserstoffatomen oder Hydroxylgruppen relativ leicht in organische Moleküle einbauen. Da die vergleichsweise lange Halbwertszeit von [18]F, die Herstellung und Anwendung [18]F-markierter Radiophannaka erleichtert, ist es das am häufigsten eingesetzte PET-Nuklid.

Somit können vor allem körpereigene Verbindungen wie Kohlenhydrate, Aminosäuren, Enzyme, Hormone oder Neurotransmitter, aber auch andere Pharmaka mit Positronenstrahlern markiert werden, ohne dass sich deren Struktur und damit deren biochemische und pharmakologische Eigenschaften wesentlich ändern. Die verabreichten absoluten Mengen der markierten Substanzen sind so gering (im mikromolaren Bereich), dass die physiologischen Konzentrationen nicht beeinflusst werden.

### Erzeugung von Positronenstrahlern

Da alle medizinisch verwendeten Positronenstrahler extrem kurze Halbwertszeiten besitzen, lassen sie sich nicht vorrätig halten, sondern müssen vor Ort individuell hergestellt werden. Weil dazu ein Teilchenbeschleuniger (häufig Zyklotron) erforderlich ist, gibt es nur wenige PET-Zentren - meist in größeren Universitätskliniken.

Am Beispiel der Produktion von [18]F-Ionen soll die so genannte Target-Reaktion erläutert werden. Hier trifft ein beschleunigtes Proton im Target auf ein [18]O-Atom vom [18]O-angereichertem Wasser. Dabei entsteht unter Emission eines Neutrons ein Fluoratom [18]F:

18/8 O + p → 18/9 F + n

Dieses ist negativ geladen und verlässt als wässrige [18]F-Fluorid-Lösung das Target. [18]Fluor spielt sicherlich die weitaus größte Rolle in der Herstellung von PET-Radiopharmaka. [18]F ist auch der einzige Positronenstrahler, der in gewissem Umfang von einem PET-Zentrum mit Zyklotron zu einer anderen nuklearlnedizinischen Einrichtung transportiert werden kann.

Dabei kann bereits das applikationsfertige Radiopharmakon ausgeliefert werden oder auch das Bestrahlungsprodukt (in der Regel [18]-Fluorid), so dass die eigentliche radiochemische Synthese dann vor Ort stattfindet.

Die Positronenstrahler [11]C, [13]N und [15]O können wegen ihrer kurzen Halbwertszeit nur für die sofortige Anwendungen im PET-Zentrum produziert werden.

### Radiochemische Synthese

In den wenigsten Fällen stellt das Radionuklid in der Form, wie es im Target des Zyklotrons entsteht, bereits das radioaktive Arzneimittel dar. Deshalb wird es anschließend in eine organochemische Verbindung (Struktur aus der organischen Chemie im Gegensatz zur anorganischen Chemie) eingebaut.

Während das in der Szintigraphie verwendete Technetium-Isotop Tc-99m meistens komplex an einen rezeptorspezifischen Trägerstoff gebunden wird, werden die PET-Nuklide durch eine kovalente Bindung in das Radiopharmakon integriert.

Hierzu bedarf es einer chemischen Synthese, die sich insofern nicht von herkömmlichen Reaktionen der präparativen Chemie unterscheidet, als radioaktive Elemente und Substanzen die gleichen chemischen Eigenschaften besitzen wie ihre nicht radioaktiven Analoga.

Da die von den Startaktivitäten ausgehende Strahlendosis aber in der Regel weit über den zulässigen Grenzwerten liegt, werden fast alle routinemäßig hergestellten PET-Radiopharmaka nicht manuell, sondern ferngesteuert mithilfe von automatischen Synthesemodulen oder durch Roboter hergestellt.

Das Funktionsprinzip aller automatischen Synthesemodule ist eigentlich immer gleich. Die Synthesemodule erlauben es, Flüssigkeiten von einem Gefäß in ein anderes zu transportieren, diese zu rühren, zu erhitzen, abzukühlen, einzuengen, zu extrahieren, (steril) zu filtrieren und abzufüllen.

Nach der Synthese erforderliche Spül- und Reinigungsvorgänge sollten ebenfalls möglichst automatisch ablaufen und schnell und einfach durchzuführen sein, so dass in kurzem Zeitabstand eine erneute Synthese in der Apparatur ablaufen kann.

Oft ist am Schluss die Aufreinigung des Produkts durch - ebenfalls automatisierte - präparative HPLC erforderlich. Schließlich wird die wässrige Lösung des Radiopharmakons durch Zusatz einer entsprechenden Menge Kochsalz(lösung) isotonisiert und über einen Sterilfilter in das Abgabegefäß gedrückt.
Zu den wichtigsten PET-Radiopharmaka zählen
- [11]C-Methionin als wichtiger Marker im Aminosäurestoffwechsel,
- 1-[11]C-Acetat zur Herzdiagnostik,
- [15]O-markiertes Wasser zur Diagnostik von Durchblutungsstörungen und
- [18]F-Fluor-DOPA zur frühzeitigen Diagnose von Morbus Parkinson und anderer dopaminerger Störungen.
- [18]F-2-Fluor-2-desoxyglucose (FDG) stellt sicherlich das wichtigste PET-Radiopharmakon dar, das vor allem in der onkologischen Diagnostik, aber auch in anderen Bereichen Anwendung findet (s. u.).

Darüber hinaus wichtig ist das Merkmal aller in der Humandiagnostik verwendeten Radiopharmaka, insbesondere PET-Radiopharmake, dass alle Substanzen lediglich eine einfache Markierung mit einem diagnostisch Wirksamen Nuklid aufweisen. Jedes Molekül kann demnach maximal ein diagnostisch nutzbares Signal entsenden.

### Nuklearmedizinische Diagnostik

Die Voraussetzung aller nuklearmedizinischer Untersuchungen ist die Applikation eines radioaktiven Stoffs, des Radiopharmakons, in den menschlichen Körper. Dieses wird meistens intravenös verabreicht und verteilt sich mit dem Blut zunächst mehr oder weniger homogen über den gesamten Körper.

Aufgrund der biologischen Funktion des Radiopharmakons oder einer spezifischen Affinität zu bestimmten Substanzen und Rezeptoren kommt es zu einer inhomogenen Umverteilung, die aufgrund der radioaktiven Strahlung durch äußere Detektoren bzw. Tomographen messbar ist und Rückschlüsse über den Funktionszustand der untersuchten Organe und Körperregionen gibt.

### Tumordiagnostik mit FDG-PET

Das weitaus am häufigsten verwendete PET-Radiopharmakon ist [18]F-2-Fluor-2-desoxyglucose, meist als FDG abgekürzt. Es handelt sich dabei um ein Glucosemolekül, das am C2 statt einer OH-Gruppe ein radioaktives [18]F-Atam trägt. Nach der intravenösen Applikation reichert es sich vor allem in Zellen mit einem erhöhten Glucose-Bedarf an.

Der Körper unterscheidet nämlich nicht zwischen "normaler" Glucose und der [18]F-markierten Fluordesoxyglucose. Da in Tumorzellen einiger Tumorarten ein erhöhter Stoffwechsel herrscht, nehmen sie mehr Glucose bzw. FDG auf als nicht entartete Zellen, so dass die FDG-PET Tumoren lokalisieren kann.

Nicht immer jedoch muss eine außergewöhnliche Konzentrierung der radioaktiven Glucose auf ein Krebsgeschehen hindeuten. Z. B. reichert sich FDG stark unspezifisch im Gehirn an, was zum einen die Untersuchung von neurologischen Fragestellungen erschwert und auch eine erhebliche Strahlenbelastung des Hirngewebes zur Folge hat.

Darüber hinaus ist die FDG-PET gestützte Diagnose nur im Falle von Tumorarten mit erhöhter Glucoseaufnahme erfolgreich und schließt somit die Lokalisierung langsam wachsender Tumorarten aus.

### Messtechnik

Die Reichweite eines emittierten Positrons im Gewebe beträgt maximal nur einige wenige Millimeter, bis es beim Zusammentreffen mit einem Elektron in zwei diametral auseinander fliegende γ-Quanten umgewandelt wird. Der ganze Prozess von der Emission des Positrons bis zur Umwandlung der Massen in die beiden γ-Quanten dauert etwa 10⁻¹⁰ Sekunden (0,1 ns).

Zum Nachweis der γ-Quanten durch die PET dient ein Koinzidenz- oder Paardetektor. Wenn jeder der beiden Einzeldetektoren, die im 180° Winkel positioniert sind, innerhalb eines sehr kurzen Zeitintervalls von etwa 10 ns ein γ-Quant definierter Energie registriert, dann schließt man daraus, dass ein Positronenzerfall irgendwo auf der Verbindungsstrecke zwischen den beiden Detektoren stattgefunden hat.

Neben Limitationen im Bereich der Detektortechnik ist die intrinsische Auflösungsgrenze durch die mittlere freie Weglänge des Positrons im umgebenden Medium (Gewebe, Gewebeflüssigkeit, Blut etc) bestimmt. Könnte man die Elektronendichte in der Umgebung des Positronenzerfalls erhöhen, so erhöht sich die Wahrscheinlichkeit der Wechselwirkung des Positrons mit einem Elektron. Somit verkürzt sich die mittlere freie Weglänge und die Auflösungsgrenze kann auf geringere Größen verbessert werden.

### Zusammenfassend weist der Stand der Technik insbesondere folgende Nachteile und Limitationen auf:

Der Stand der Technik beschreibt nur diagnostische Mittel aus der Menge der Substanzen der organischen Chemie. Es werden zur Herstellung der organischen Substanzen zeitaufwändige, oft mehrstufige Synthese- und Aufreinigungsschritte benötigt, die dazu führen, dass aufgrund der geringen Halbwertszeiten der Nuklide die relative Ausbeute an diagnostisch wirksamen Produkt mit zunehmender Herstellungszeit geringer wird. Das meist genutzte PET-Diagnostikum, FDG, reichert sich überdies stark unspezifisch im Gehirn an, was zum einen die Untersuchung von neurologischen Fragestellungen erschwert und auch eine erhebliche Strahlenbelastung des Hirngewebes zur Folge hat. Des Weiteren ist die FDG-PET gestützte Diagnose nur im Falle von Tumorarten mit erhöhter Glucoseaufnahme erfolgreich und schränkt somit die Lokalisierung langsam wachsender Tumorarten stark ein. Da die gemäß dem Stand der Technik in der Humandiagnostik genutzten Radiopharmaka, insbesondere PET-Radiopharmaka, lediglich eine einfache Markierung mit einem diagnostisch Wirksamen Nuklid aufweisen, kann jedes Molekül demnach maximal ein diagnostisch nutzbares Signal entsenden. Darüber hinaus haben die organischen PET-Radiopharmaka eine ähnliche Elektronendichte wie das umliegende Gewebe. Die räumliche Auflösung kann also nicht verbessert werden.

Die der vorliegenden Erfindung zugrunde liegende Aufgabe bestand somit darin, neue medizinisch, insbesondere diagnostisch, nutzbare Mittel zu finden, die die zuvor geschilderten Nachteile des Standes der Technik vermeiden.

Gegenstand der vorliegenden Erfindung ist somit ein pharmazeutisches Mittel enthaltend eine partikuläre anorganische Matrix, die neben der natürlichen Isotopenverteilung der Strukturtypen-bildenden Elemente der Anionen bzw. Kationen Anteile an Positronen-emittierenden Nukliden enthält, wobei die Anzahl der Positronen-emittierenden Nuklide pro partikulärer anorganischer Matrix vorzugsweise größer gleich 1 ist.

Die möglichen Strukturtypen der partikulären anorganischen Matrix können sowohl Kristallstrukturen als auch amorphe Strukturen oder Mischungen aus beiden umfassen. Der Begriff schließt sowohl Kolloide, Sole, Suspensionen, Nanosuspensionen, amorphe und kristalline Partikel ein.

Bevorzugt ist die Anzahl der medizinisch, insbesondere diagnostisch, nutzbaren, also der Positronen-emittierenden Nuklide pro partikulärer anorganischer Matrix 1 bis 10 000 000 und besonders bevorzugt 5 bis 1 000 000, sowie ganz besonders bevorzugt 10 bis 100 000.

Die erfindungsgemäßen Partikel (im folgenden auch sinngemäß für partikuläre anorganische Matrix) bestehen maßgeblich (siehe auch Definition der Strukturtypen) aus in Wasser und physiologischen Flüssigkeiten schwerlöslichen anorganischen Verbindungen beispielsweise aus Topas, (Al₂F₂)[SiO₄] und Chiolith, Na[Al₃F₄], bevorzugt Wavellit, Al₃(PO₄)₂(OH, F)₂, Calciumcarbonat, CaCO₃, Maghemit, γ-Fe₂O₃, besonders bevorzugt Zeolithe, allg. Formel Mₙ[(AlO₂)ₓ(SiO₂)_{y}] (M= Metall z.B. Na), Magnetit, Fe₃O₄, und Bariumsulfat, BaSO₄, sowie ganz besonders bevorzugt Galliumphosphat, GaPO₄, Apatit bzw. Fluorhydroxylapatit, Ca₅(PO₄)₃(OH, F) = 3Ca₃(PO₄)₂ * Ca(OH, F)₂, und Flußspat, CaF₂.

Bevorzugtermassen sind die Positronen-emittierenden Nuklide ausgewählt aus der Gruppe umfassend [15]O, [30]P, [13]N, [65]Ga,[11]C, [131]Ba, [26]Al, und [68]Ga und [18]F. Die medizinisch, insbesondere diagnostisch, nutzbaren Nuklide für die erfindungsgemäßen Partikel sind somit [15]O, [30]P, [13]N, bevorzugt [65]Ga,[11]C, besonders bevorzugt [131]Ba, [26]Al, sowie ganz besonders bevorzugt [68]Ga bzw. [18]F. Darüber hinaus können alle dem Fachmann bekannten medizinisch nutzbaren Isotope verwendet werden, um erfindungsgemäße Partikel herzustellen (Ein Auszug einiger Isotope ist im Anhang zu finden).

Der Durchmesser der erfindungsgemäßen Partikel kann in einem Bereich von 0,1nm bis 100µm liegen, vorzugsweise 1nm bis 10µm, besonders bevorzugt 1nm bis 1µm.

In einer bevorzugten Ausführungsform des erfindungsgemäßen pharmazeutischen Mittels sind die erfindungsgemäßen Partikel in einer pharmazeutischen Hülle.

Erfindungsgemäß ist die Hülle ausgewählt aus der Gruppe umfassend: ein synthetisches Polymer oder Copolymer, eine Stärke oder ein Derivat davon, ein Dextran oder ein Derivat davon, ein Cyclodextran oder ein Derivat davon, eine Fettsäure, ein Polysaccharid, ein Lecithin oder ein Mono-, Di- oder Triglycerid oder ein Derivat davon oder Gemische davon.

Das synthetische Polymer oder Copolymer kann ausgewählt sein aus der Gruppe umfassend Polyoxyethylensorbitanester, Polyoxyethyle und Derivate davon, Polyoxypropyle und Derivate davon, nichtionische oberflächenaktive Substanzen, Polyoxylstearate (35-80), Polyvinylalkohole, polymerisierte Sucrose, Polyhydroxyalkylmethacrylamide, Milchsäure und Glycolsäure-Copolymere, Polyorthoester, Polyalkylcyanoacrylates, Polyethylenglycol, Polypropylenglycol, Polyglycerole, polyhydroxylierte Polyvinylmatrices, Polyhydroxaethylaspartamide, Polyaminosäuren, Styrol- und Maleinsäure-Copolymere, Polycaprolactone, Carboxypolysaccharid, und Polyanhydridn.

Das Stärkederivat kann ausgewählt sein aus der Gruppe umfassend Stärke-2-hydroxymethylether und Hydroxyethylstärke.

Das Dextran oder Derivat davon kann ausgewählt sein aus der Gruppe umfassend galactosyliertes Dextran, lactosyliertes Dextran, aminiertes Dextran, Dextran mit SH-Gruppen, Dextran mit Carboxylgruppen, Dextran mit Aldehydgruppen, biotinyliertes Dextran.

Das Cyclodextrin kann ausgewählt sein aus der Gruppe umfassend beta-Cyclodextrin und Hydroxypropylcyclodextrin.

Die Fettsäure kann ausgewählt sein aus der Gruppe umfassend Natriumlaurylsulfat, Natriumstearat, Stearinsäure, Sorbitanmonolaurat, Sorbitanmonooleate, Sorbitanmonopalmitat und Sorbitanmonostearat.

Eine andere bevorzugte Ausführungsform des erfindungsgemäßen pharmazeutischen Mittels besteht darin, dass die anorganischen Partikel an target-spezifische Liganden angekoppelt sind. Der Ligand kann ausgewählt sein aus der Gruppe umfassend Proteine, Antikörper, Antikörperfragmente, Peptide, Aptamere, Darpine und anderer Moleküle, die eine hohe Affinität zu krankheitsspezifischen Rezeptoren besitzen. Entsprechende Moleküle mit Rezeptoraffinität sind dem Fachmann bekannt.

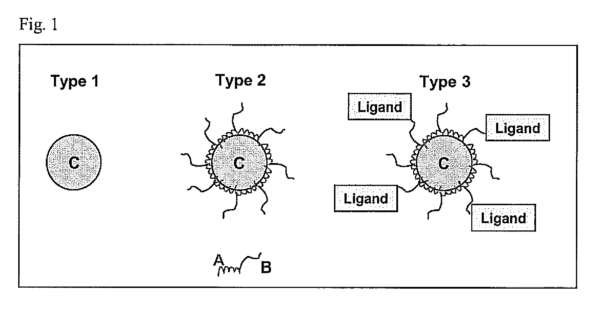

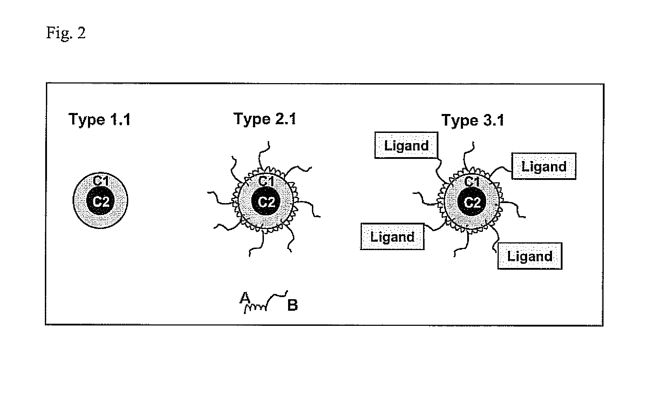

Drei schematisierte Strukturtypen bzw. Ausführungsformen der erfindungsgemäßen Partikel sollen hier beschrieben werden (siehe Figur 1 und 2):

### Beschreibung der Figuren 1 und 2.

**Figur 1** **Typ 1** beschreibt erfindungsgemäße Partikel aus der oben beschriebenen Gruppe der schwerlöslichen anorganischen Verbindungen (C), die stabilisatorfrei koagulationsgehemmt sind. Hier tragen adsorbierte Wassermoleküle oder/und die elektrische Aufladung der Partikeloberfläche zur Stabilisierung der Partikel bei (auch Sol genannte Systeme).

**Figur 1** **Typ 2** sind erfindungsgemäße Partikel, die mit grenzflächenaktiven Molekülen gegen Aggregation stabilisiert sind. Neben den gängigen i.v. applizierbaren Stabilisatoren wie beispielsweise Poloxamere, können auch speziell maßgeschneiderte Blockcopolymere eingesetzt werden. Hier sind z.B. so genannte doppelhydrophile A-Block-B-Copolymere zu nennen, deren Block A koodinierende/stabilisierende Fähigkeiten besitzt und der zweite Block B die Partikel vor Aggregation schützt und oft aus Polyethylenglycol (PEG) besteht. Darüber hinaus können die erfindungsgemäßen Partikel mit weiteren Materialen beschichtet werden, z.B. mit klassischen Beschichtungsmaterialien wie Kohlenhydraten, Polyethylenglycolen, Polyacryl- oder-mekhacylsäuren, Fettsäuren, Silica oder/und Silan etc..

**Figur 1** **Typ 3** zeigt eine weitere Ausführungsform der erfindungsgemäßen Partikel mit zielspezifischer Partikelstruktur, die durch zusätzliches Ankoppeln von spezifischen Liganden erhalten werden. Diese Liganden können beispielsweise Antikörper, deren Fragmente, Peptide, Aptamere, Darpine oder andere kleine Moleküle sein, die eine hohe Affinität zu krankheitsspezifischen Rezeptoren besitzen. Die Liganden können sowohl über chemische Modifizierung der zur Stabilisierung verwendeten Substanzen gekoppelt werden als auch direkt am erfindungsgemäßen Partikel verankert sein.

Zusätzlich kann der Kern der erfindungsgemäßen Partikel aus zwei verschiedenen Materialen bestehen (Kern-Schale-Struktur; **Figur 2** **Typ 1.1, 1.2, 1.3**). Dabei kann in einer bevorzugten Ausführungsform das innere C2 (oder äußere C1) aus einem weiteren diagnostisch nutzbaren Mittel bestehen. Beispielsweise können Magnetite (wie Resovist bzw. Supravist, Schering AG Deutschland) als Nukleationskeime genutzt werden und die oben beschriebenen schwerlöslichen anorganischen Verbindungen auf diesen gefällt bzw. aufgebracht werden. In einer weiteren Ausführungsform kann die Schale der erfindungsgemäßen Partikel vom Typ 1.1, 1.2 und 1.3 aus jeder pharmazeutisch annehmbaren Hülle bestehen.

Ebenfalls ist Gegenstand der vorliegenden Erfindung eine pharmazeutische nutzbare Zusammensetzung bei der mindestens 0,001% der in der Zusammensetzung enthaltenden Partikel die erfindungsgemäßen Partikel sind. Das heißt, unter Umständen reicht schon ein geringer Anteil der in dem erfindungsgemäßen pharmazeutischen Mittel enthaltenen Partikel in einer pharmazeutisch nutzbaren Zusammensetzung aus, um diese Zusammensetzung diagnostisch oder therapeutisch anwendbar zu machen.

Vergleicht man die erfindungsgemäßen Partikel mit FDG, dem Stand der Technik des PET-Tracermarktes, so können eine Reihe von Vorteilen beschrieben werden, die sich direkt aus dem partikulären Charakter der erfindungsgemäßen Partikel ableiten lassen.
1. Einfache Herstellung: Zur Herstellung der anorganischen Nanopartikel benötigt man im Gegensatz zu FDG keine organische Syntheseschritte und Aufreinigung. Die erfindungsgemäßen anorganischen partikulären PET-Tracer bilden sich spontan bei Raumtemperatur einzig durch Zugabe des Isotops, z.B.[18]F zu Hydroxylapatit, und Mischen der im so genannten "cold-kit" vorformulierten Edukte. Das Distribution Center bzw. der Nuklearmediziner spart also Ressourcen.
2. Kurze Herstellzeit: Die Herstellungszeit einer Menge erfindungsgemäßer Partikel Formulierung ist mindestens um den Faktor 10 geringer als die benötigte Zeit zur Synthese und Aufreinigung von FDG gleicher Aktivität, d.h. bei gleicher Lieferzeit kommt mehr wirksames Produkt beim Kunden an.
3. Längere Verwendbarkeit: Die Halbwertszeit der Isotope, beispielsweise [18]F, ist zwar eine Naturkonstante (t1/2 ca. 110min), doch ein erfindungsgemäßes Partikel in einer diagnostisch nutzbaren Formulierung kann je nach Partikelgröße, die durch den Herstellungsprozess kontrolliert wird, bis zu mehreren hunderttausend [18]F Atome enthalten. Dies bedeutet, dass jedes pharmakologisch wirksame Partikel auch hunderttausendfach länger in der PET Bildgebung aktiv ist als ein einfach [18]F markierter Tracer. Dies ist insbesondere dann von kommerziellem Interesse, wenn 1:1 Konjugate von teuren Biomolekülen wie Antikörpern und PET-Isotopen zur Anwendung kommen. Auch können so markierte Zellen länger mittel PET Bildgebung im Organismus verfolgt werden.
4. Passives Targeting: Gesunde Blutgefässe haben ein geschlossenes Endothel. Sie sind dicht und halten größere Moleküle wie z.B. Albumin oder auch Nanopartikel zurück in der Blutbahn. Das Endothel von Blutgefässen in Tumoren hat Löcher, durch diese kleine erfindungsgemäße Partikel leicht aus der Blutbahn ins Gewebe diffundieren können. Da überdies der Lymphtransport im Tumorgewebe vermindert ist, reichern sich die erfindungsgemäßen Partikel im Tumor an - allein durch die richtige nanoskalige Größe.
5. Spezifität: Nanopartikel eignen sich zum zellassoziierten Targeting, d.h. die Partikel werden je nach Größe, Oberflächenstruktur und -ladung vornehmlich von Zellen des RES aufgenommen. Dabei können im Blut zirkulierende Makrophagen nach Injektion der Formulierung direkt mit erfindungsgemäßen Partikeln markiert werden. Dies ermöglicht die Bildgebung von bspw. entzündlichen Prozessen, die stets von vermehrter Makrophagenaktivität begleitet werden.
6. Plattform für molekulare Bildgebung: Neben den Möglichkeiten des passiven und zellassoziierten Targetings bieten die erfindungsgemäßen Partikel eine weitere zielgerichtete Adressierung der anorganischen Partikel. Durch die Kopplung von krankheitsspezifischen Liganden an die Oberfläche der erfindungsgemäßen Partikel wird ein aktives Targeting von Rezeptoren z.B. auf Zelloberflächen ermöglicht.
7. Geringere Strahlenbelastung im Gehirn: Im Gegensatz zu FDG, Stand der Technik, reichern sich erfindungsgemäße Partikel nicht unspezifisch im Gehirn an. Dies reduziert zum einen die Strahlenbelastung des Gehirns und zum anderen wird eine Untersuchung von neurologischen Fragestellungen möglich.
8. Detektion sehr kleiner Herde: Neben weiteren Faktoren hängt die räumliche Auflösung der PET intrinsisch von der mittleren freien Weglänge der Positronen im Gewebe ab (ca. 3mm). Da beispielsweise ein [18]F-Isotap des FDG frei im Gewebe zerfällt, kann die Auflösung nicht unter diese physikalische Grenze gebracht werden. Demgegenüber sind die Isotope der erfindungsgemäßen Partikel fest in ein Kristallgitter eingeschlossen. Dies erhöht die Wechselwirkungswahrscheinlichkeit der Positronen mit Elektronen und reduziert die mittlere freie Weglänge. Eine Verbesserung der räumlichen Auflösung der PET kann erreicht werden.
9. Erweitertes Einsatzspektrum: Die erfindungsgemäßen Partikel haben eine ähnliche oder gar höhere Elektronendichte wie Knochen, d.h. eine Anhäufung von Partikeln kann direkt im CT abgebildet werden. Eine nichtradioaktive Formulierung erfindungsgemäßer Partikel (beispielsweise bestehend aus CaF₂, das natürlich häufigste Fluorisotop, [19]F, ist nicht radioaktiv) eignet sich zur Bildgebung in der F-MRT.
10. Multimodale Bildgebung: Erfindungsgemäße Partikel insbesondere der Ausführungsform 1.1, 1.2 und 1.3 (siehe Fig. 2) eignen sich zur multimodalen Bildgebung, da diese neben dem Positronenemitter eine weitere diagnostisch nutzbare Substanz enthalten.

### Mögliche Herstellvarianten

Darüber hinaus betrifft die Erfindung Verfahren zur Herstellung von pharmazeutischen Mitteln enthaltend eine partikuläre anorganische Matrix, die neben der natürlichen Isotopenverteilung der Strukturtypen-bildenden Elemente der Anionen bzw. Kationen Anteile an Positronen-emittierenden Nukliden enthält, wobei die Anzahl der Positronen-emittierenden Nuklide pro partikulärer anorganischer Matrix vorzugsweise größer gleich 1 ist.

Somit ist Gegenstand der vorliegenden Erfindung auch ein Verfahren zur Herstellung eines erfindungsgemäßen pharmazeutischen Mittels umfassend mindestens einen der folgenden Schritte:
(i) Mischen mindestens zweier Lösungen löslicher Salze gemäß einer Fällungsreaktionsgleichung, wobei mindestens ein Salz mit Positronen-emittierenden Nukliden angereichert ist und wobei die Kationen des einen Salzes mit den Anionen des anderen Salzes ausfallen und wobei der Positronen-emittierende Anteil Teil der gefällten Verbindung ist und somit eine partiküläre anorganische Matrix bildet, die neben der natürlichen Isotopenverteilung der Strukturtypen-bildenden Elemente der Anionen bzw. Kationen Anteile an Positronen-emittierenden Nukliden enthält.
(ii) Bildung einer Suspension gemäß einer Fällungsreaktionsgleichung enthaltend die partikuläre anorganische Matrix, die neben der natürlichen Isotopenverteilung der Strukturtypen-bildenden Elemente der Anionen bzw. Kationen Anteile an Posüronen-emittierenden Nukliden enthalten.
(iii) Mischen mindestens zweier Lösungen löslicher Salze gemäß einer Fällungsreaktionsgleichung zur Bildung einer anorganischen Matrix und nachträglicher Zugabe der Positronen-emittierenden Nuklide in anionischer bzw. kationischer Form.

Im Rahmen der vorliegenden Erfindung kann eine Zugabe von Stabilisatoren und/oder Ligand tragenden Stabilisatoren erfolgen. Im Rahmen der vorliegenden Erfindung kann eine zusätzliche Nutzung von nichtradioaktiven Isotopen erfolgen. Gegebenenfalls kann die Herstellung und Nutzung von Precurser als Nukleationskeime für das erfindungsgemäße Verfahren erfolgen. Das erfindungsgemäße Verfahren kann ebenfalls durchgeführt werden durch die Nutzung von Austauschprozessen, die beispielsweise einen Austausch von OH⁻ - Ionen gegen [18]F⁻-Ionen in der Partikelstruktur ermöglichen. Bei dem erfindungsgemäßen Verfahren kann eine Teilchengrößenkontrolle durch die Variation der Konzentrationsverhältnisse der Edukte (Ausgangsstoffe) erfolgen. Dem erfindungsgemäßen Verfahren können sich Schritte anschließen zur Modifizierung der Oberflächeneigenschaften durch Wahl der pharmazeutischen Hülle. Dem Verfahren können sich Schritte anschließen zur Kopplung von Liganden durch dem Fachmann bekannte physikalische und chemische sowie biochemische Verfahren. Bei dem erfindungsgemäßen Verfahren kann die Isotonie der Formulierung eingestellt werden. Bei dem erfindungsgemäßen Verfahren kann gegebenenfalls Sterilisation, Autoklavierung oder Sterilfiltration durchgeführt werden. Bei dem erfindungsgemäßen Verfahren können in einem weiteren Schritt anorganische Partikel eines vorbestimmten Gesamtpartikeldurchmessers ausgewählt werden.

Im Folgenden werden bevorzugte Ausführungsformen des erfindungsgemäßen Verfahrens weiterführend beschrieben.
*(a) Generelle Herstellung:* Die oben beschriebenen Verbindungen können durch einfaches Mischen zweier wässriger Lösungen der wasserlöslichen Salze mit natürlicher Isotopenverteilung und benötigter Menge an Positronen emittierenden Edukten hergestellt werden. Dies kann beispielsweise durch eintropfen unter Rühren oder Schütteln, in speziellen Mischkammern oder auch durch Nutzung von Mikromischern, die aus der Mikrosystemtechnik bekannte sind, erfolgen. Die gängigen Module zur speziellen Synthese von Radiopharmaka können auch genutzt werden, da diese die notwendigen Misch- und Rührelemente im Aufbau enthalten. Die Herstellung der hier beschriebenen Partikel ist jedoch so einfach, dass auch ein handliches Mischsystem, in dem Edukte, Mischkammer und Applikationsspritze vereint sind, entwickelt werden kann. Die partikuläre Suspension entsteht sofort nach Mischung der Lösungen und die Herstellungszeit liegt im Bereich von Sekunden.
*(b) Gegebenfalls Herstellung verschiedener Strukturtypen:* Die Mischung kann ohne Zugabe von Stabilisator erfolgen (Strukturtyp 1 und 1.1), mit Stabilisator (Strukturtyp 2 und 2.1) oder mit Ligand tragendem Stabilisator (Strukturtyp 3 und 3.1). Auch eine Copräzipitation mit Liganden oder modifizierten Liganden ist durchführbar.
*(c) Gegebenfalls Zusätzliche Nutzung von nichtradioaktiven Isotopen:* Die Herstellung der Partikel kann aus den löslichen Verbindungen direkt mit den radioaktiven Isotopen, beispielsweise [18]F⁻, das in wässriger Lösung durch Kernreaktion im Cyclotrons anfällt, erfolgen. Durch Nutzung von nicht radioaktiven Isotopen wie z.B. [19]F⁻ kann die die Prozessführung optimiert werden.
*(d) Gegebenfalls Herstellung und Nutzung von Precurser:* Eine weitere Herstellvariante liegt in der Nutzung von Precursern, die als Nukleationskeime dienen können, so dass eine Kern-Schale Struktur entsteht. Als Nukleationskeim kann die Zielverbindung selbst - allerdings mit der natürlichen Isotopenverteilung - oder auch ein zweites Kontrastmittel wie z.B. Magnetit- oder Maghemitpartikel eingesetzt werden.
*(e) Gegebenfalls Herstellung durch Nutzung von Austauschprozessen:* Am Beispiel von Hydroxylapatit kann eine fertige Formulierung der erfindungsgemäßen Strukturtypen ganz einfach mit wässriger [18]F- Lösung umgesetzt werden. Die [18]F⁻ Ionen tauschen OH- Ionen in der Hydroxylapatitstruktur aus, und es entsteht partikuläres [18]F-Fluorhydroxylapatit, Ca₅(PO4)₃(OH, [18]F), ohne dass die Überstruktur gestört ist.
*(f) Gegebenfalls Teilchengrößenkontrolle:* Die Größe der Nanopartikel kann durch die Konzentrationsverhältnisse der Edukte (Ausgangsstoffe) kontrolliert werden.
*(g) Gegebenfalls Modifizierung der Oberflächeneigenschaft:* Durch den Einsatz von unterschiedlichen Stabilisatoren können die Oberflächeneigenschaften bestimmt werden.
*(h) Gegebenfalls Kopplung von Liganden:* Das Anfügen von Liganden durch gängige physikalische und chemische sowie biochemische Verfahren.
*(i) Gegebenfalls Einstellung der Isotonie:* Durch die Verwendung der jeweils löslichen Cl- bzw. Na-Verbindungen - oder anderer üblicher Stoffe zur Isotonierung - kann gleich auf die spätere Isotonie der resultierenden Formulierung Rücksicht genommen werden.
*(j) Gegebenfalls Sterilisation*/*Autoklavierung*/*Sterilfiltration:* Die Formulierungen - Precurser bzw. fertige Zubereitung - können mit üblichen Verfahren sterilisiert werden.

Die beschriebenen Ausführungsformen und Verfahren zur Herstellung zeigen auf, dass die erfindungsgemäßen Partikel in ihren Eigenschaften wie beispielsweise Zusammensetzung, Isotopengehalt, Größe und Oberflächeneigenschaften in verschiedenen Formulierungen bereitgestellt werden können, die so gezielt auf die pharmakologischen Anforderungen abgestimmt werden können.

### Verwendung der erfindungsgemäßen Partikel

Die erfindungsgemäßen pharmazeutischen Mittel werden in der Medizin, vorzugsweise als diagnostisches Mittel, in-vitro und in-vivo eingesetzt. Besonders bevorzugt ist die Verwendung in der Positronen-Emissions-Tomographie (PET) und auch PET-CT bildgestützten Diagnose. Die erfindungsgemäßen Partikel können sowohl in der pharmakologischen Forschung und in der Veterinärmedizin als auch in der Humanmedizin eingesetzt werden. Für die diagnostische Anwendungen wird einem Patienten die wirksame Dosis verabreicht. Die Verabreichung erfolgt vorzugsweise intravenös. Die Verteilung der erfindungsgemäßen Partikel im Körper wird dann nach vorbestimmten Zeiten durch PET bzw. PET-CT oder gegebenenfalls durch andere geeignete Methoden bestimmt. Entsprechende Verfahren sind dem Fachmann bekannt. Die erfindungsgemäßen pharmazeutischen Mittel werden ebenfalls in der Iocoregionalen Therapie eingesetzt.

Mögliche Indikationen und Nutzungsmechanismen werden im folgenden beispielhaft beschrieben.

### Verwendung der erfindungsgemäßen Partikel analog zu Kolloiden für die MRT z.B. Resovist und Supravist (Schering AG, Deutschland)

Der Einsatz der erfindungsgemäßen Partikel in allen Resovist, z.B. Untersuchung der Leber, und Supravist, z.B. Angiographie, Indikationen ist möglich.

### Verwendung der erfindungsgemäßen Partikel analog zu Kolloiden für die SPECT z.B. NanoCis (CisBio, Frankreich)

Der Einsatz der erfindungsgemäßen Partikel in allen NanoCis/NanoColl Indikation, z.B. Sentinall Lymphknoten Detektionen, ist prinzipiell möglich. Auch sind die Kern-Schale Strukturtypen mit Magnetit im Kern für kombinierte Imagingverfahren im Bereich der Sentinell Lymphknoten Detektion nutzbar.

### Verwendung der erfindungsgemäßen Partikel zum "Passive Targeting"

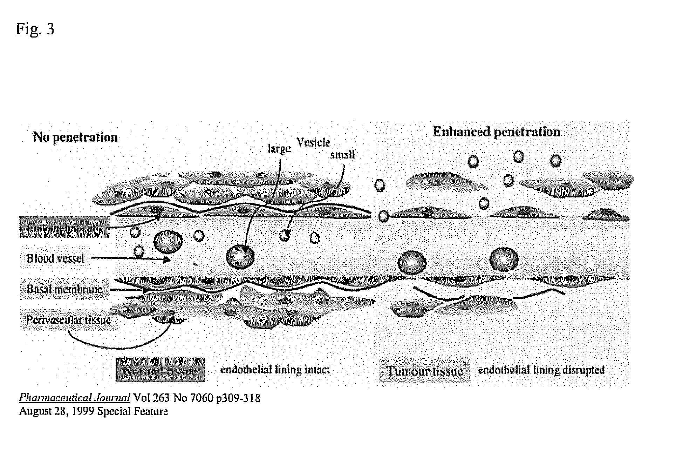

Eine Kontrolle der Größe und Oberflächeneigenschaften erlaubt das Maßschneidern von Nanopartikeln für das passive Targeting von Tumorgewebe. Der Mechanismus des Passive Targeting wird in Figur 3 schematisch dargestellt.

### Beschreibung der Figur 3.

Gesunde Blutgefässe haben ein geschlossenes Endothel. Sie sind dicht und halten größere Moleküle wie z.B. Albumin oder auch Nanopartikel zurück in der Blutbahn. Das Endothel von Blutgefässen in Tumoren hat Löcher, durch diese kleine erfindungsgemäße Partikel leicht aus der Blutbahn ins Gewebe diffundieren können. Da überdies der Lymphtransport im Tumorgewebe vermindert ist, reichern sich die erfindungsgemäßen Partikel im Tumor an - allein durch die richtige nanoskalige Größe.

Das spezifische Milieu im Tumorgewebe kann darüber hinaus dafür sorgen, dass die erfindungsgemäßen Partikel dort andere Eigenschaften annehmen, die diagnostisch nutzbar sind.

### Verwendung der erfindungsgemäßen Partikel für das zellassoziierte Targeting

Die erfindungsgemäßen Partikel werden je nach Größe, Oberflächenstruktur und -ladung vornehmlich von Zellen des RES aufgenommen. Dabei können im Blut zirkulierende Makrophagen nach Injektion der Formulierung direkt mit erfindungsgemäßen Partikeln markiert werden. Dies ermöglicht die Bildgebung von bspw. entzündlichen Prozessen, die stets von vermehrter Makrophagenaktivität begleitet werden.

### Verwendung der erfindungsgemäßen Partikel für "Active Targeting"

Mit Hilfe von spezifischen Liganden können gezielt Gewebestrukturen erkannt werden. Beispielsweise eignet sich Anti-L-Selectin zur Darstellung von Lymphknoten. ICAM und VCAM sind spezifische Entzündungsmarker, die auch im Falle der Detektion von MS eine große Rolle spielen. Darüber hinaus eignet sich E-Selectin zur Erkennung von Entzündungsherden und auch vielen Tumortypen. EDB-Fibronektin ist angionesespezifisch und zur Darstellung von Brustkrebs kann ein anti-CD105 (Endoglin) Antikörper genutzt werden. Weitere nutzbare Liganden und Rezeptoren sind dem Fachmann bekannt.

Da die Herstellung der erfindungsgemäßen Partikel unkompliziert ist, und das Wechseln der spezifischen Liganden insbesondere über die Stabilisatoren erfolgen kann, dienen die erfindungsgemäßen Partikel als flexible Plattformtechnologie.

### Verwendung der erfindungsgemäßen Partikel zur Stammzellen Bildgebung (Steem-Cell Tracking)

Stammzellenmarkierungen sind mit den erfindungsgemäßen Partikeln möglich.

### Verwendung der erfindungsgemäßen Partikel zur Überwindung der Blut Hirn Schrank (BBB Delivery)

Mit Hilfe von Literatur bekannten Delivery Strategien (z.B. Pathfinder Technology) ist die Überschreitung Blood Brain Barrier möglich.

Somit ist auch Gegenstand der gegenwärtigen Erfindung ein erfindungsgemäßes pharmazeutisches Mittel zur Diagnose mittels Positronen-Emissions-Tomographie einer Erkrankung ausgewählt aus der Gruppe umfassend proliferative Erkrankungen, inflammatorische Erkrankungen, Autoimmunerkrankungen, Erkrankungen des Verdauungstrakts, Arteriosklerose, Schlaganfall, Infarkt, krankhafte Veränderungen des Blutgefäßsystems, des Lymphsystems, der Bauchspeicheldrüse, der Leber, der Niere, des Gehirns und der Blase, sowie Erkrankungen der elektrischen Reizweiterleitung und neurodegenerative Erkrankungen. Dabei kann die proliferative Erkrankung ausgewählt sein aus der Gruppe bestehend aus einem Tumor, einer Präcancerose, einer Dysplasie, einer Endometriose und einer Metaplasie. Dabei kann die Autoimmunerkrankung ausgewählt sein aus der Gruppe bestehend aus rheumatoider Arthritis, inflammatorischer Darmerkrankung, Osteoarthritis, neuropathischen Schmerzen Alopecia areta, Psoriasis, psoriatische Arthritis, akute Pancreatitis, Allograftabstoßung, Allergien, allergische Entzündungen der Lunge, Multiple Sclerosis, Alzheimer, Morbus Crohn, und systemischer Lupus erythematosus.

Somit ist auch Gegenstand der gegenwärtigen Erfindung ein erfindungsgemäßes pharmazeutisches Mittel zur locoregionalen Therapie einer Erkrankung ausgewählt aus der Gruppe umfassend proliferative Erkrankungen, inflammatorische Erkrankungen, Autoimmunerkrarlkungen, Erkrankungen des Verdauungstrakts, Arteriosklerose, Schlaganfall, Infarkt, krankhafte Veränderungen des Blutgefäßsystems, des Lymphsystems, der Bauchspeicheldrüse, der Leber, der Niere, des Gehirns und der Blase, sowie Erkrankungen der elektrischen Reizweiterleitung und neurodegenerative Erkrankungen. Dabei kann die proliferative Erkrankung ausgewählt sein aus der Gruppe bestehend aus einem Tumor, einer Präcancerose, einer Dysplasie, einer Endometriose und einer Metaplasie. Dabei kann die Autoimmunerkrankung ausgewählt sein aus der Gruppe bestehend aus rheumatoider Arthritis, inflammatorischer Darmerkrankung, Osteoarthritis, neuropathischen Schmerzen Alopecia areta, Psoriasis, psoriatische Arthritis, akute Pancreatitis, Allograftabstoßung, Allergien, allergische Entzündungen der Lunge, Multiple Sclerosis, Alzheimer, Morbus Crohn, und systemischer Lupus erythematosus.

Weiterhin wird die Erfindung durch die nachfolgenden Beispiele erläutert:

### Ausführliches Beispiel 1: Nanopartikuläres Calciumfluorid, CaF₂, als PET-Diagnostikum

### 1.1 Allgemeine Beschreibung

CaF₂ fällt als schwerlösliche Verbindung (Löslichkeit von Flußspat: 17mg/L bei 20 °C und 16mg/L bei 18°C) nach Mischung der wasserlöslichen Verbindungen CaC1₂ und NaF (mit KF, NH₄F oder HF kann auch eine erfolgreiche Herstellung durchgeführt werden) als Bodensatz oder auch nach Mischung geeignet geringer Konzentrationsverhältnisse Kolloiddispers ("Nanopartikel") aus.

Fällungsreaktionsgleichung:

CaCl₂ + 2 F⁻ → CaF₂↓ + 2 Cl⁻

In Gegenwart von geeigneten Stabilisatoren, beispielsweise Poloxamer F68, werden auch bei Mischung weit höherer Konzentrationsverhältnisse Nanopartikel gebildet. Calcophile-Stabilisatoren wie Blockcopolymere vom Typ Polyacrylsäure-block-PEG, Phosphat-funktionalisierte-Polymere-block-PEG, Phosphonat-funktionalisierte-Polymere-block-PEG sind dafür besonders gut geeignet.

Das lässt sich anhand der "niedermolekularen" Löslichkeitsprodukte ableiten:

| | |
|---|---|
| Ca-Carboxylat | pKL(25°C)= 8.1 |
| Ca-Phosphat | pKL(25°C) = 29.5 |
| Ca-Phosphonat | pKL(25 °C)= 52 |

Eine chemische Kopplung der Stabilisatoren zu zielspezifischen Liganden wie Antikörper, deren Fragmente, Peptide etc. vom Typ *Ligand-PEG-block-calcophiles-Polymer* liefert Nanopartikel, die zielgerichtet an entsprechende Targets im Patienten binden können.

[18]F⁻ verhält sich chemisch wie [19]F⁻. Für ein PET-Diagnostikum wird [18]F⁻ beispielsweise als H[18]F oder Na[18]F-Lösung der benötigten Radioaktivität der Na[19]F-Lösung zugegeben, gemischt und zur Fällung gemäß der Fällungsreaktion eingesetzt.

Das Nuklid [18]F hat eine Halbwertszeit von ca. t1/2=110 min. Es wird nach folgender Target Reaktion im Cyclotron hergestellt:

| Target Material | Target Reaktion | Produkt | Nuklid |
|---|---|---|---|
| [18]O-H₂O ... | [18]O→(p,n)→[18]F ... | [18]F⁻ | [18]F |

[18]F⁻ fällt als HF in wässriger Lösung an. Die Fluoridkonzentration liegt dabei je nach Prozessführung und Bauart des Cyclotrons in der Regel zwischen 50 und 400 GBq/µmol.

### 1.2 Durchführung

### 1.2.1 Tensidfreie CaF₂ Nanopartikel

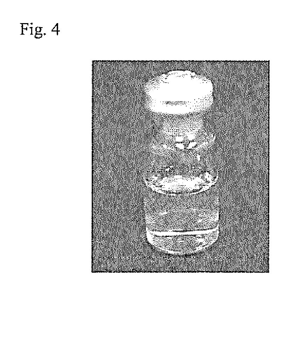

Die entsprechende Menge [18]F⁻ -Lösung wird mit nicht radioaktiver Kaliumfluorid (KF)-Lösung gemischt, so dass 1mL einer wässrigen KF-Lösung der Konzentration 0.1 mol/L entsteht. 1 mL der wässrigen KF-Lösung (0.1mol/L) werden mit 1mL einer wässrigen CaCl₂ -Lösung der Konzentration 0.05 mol/L in einem Vial durch schütteln gemischt. Die CaF₂ Nanopartikel bilden sich sofort, was man an der leichten opaleszens der erstandenen Dispersion erkennen kann (Figur 4).

Die Bestimmung der Teilchengrößenverteilung mit Dynamischer Lichtstreuung (DLS) liefert eine mittlere Partikelgröße von 71.5 nm (intensitätsgewichtet).

Die Wiederholung des Experiments liefert eine Partikelgröße von 81.7 nm.

Mit Hilfe der Dünnschichtchromatographie (Laufmittel: Methanol oder Methylethylketon) und einem geeigneten Detektor kann gezeigt werden, dass mehr als 90% der eingesetzten Radioaktivität in den erfindungsgemäßen Partikeln gebunden ist und auf der Basislinie zurück bleibt (Rf=0).

In einer zweiten Versuchreihe wird anstelle der KF-Lösung eine Natriumfluorid (NaF)-Lösung (0.1mol/L) mit 1mL einer wässrigen CaCl₂ -Lösung (0.05 mol/L) gemischt. Auch hier bilden sich CaF₂ Nanopartikel und die Dispersion erscheint opaleszent. Die Teilchengrößenbestirrlmung mit DLS liefert hier 75.9 nm. Auch hier sind mehr als 90% der eingesetzten Radioaktivität in den erfindungsgemäßen Partikeln gebunden.

### 1.2.2 Tensid stabilisierte CaF₂ Nanopartikel

1mL einer wässrigen KF-Lösung (0.1 mol/L) mit gewünschter Radioaktivität werden mit 1mL einer wässrigen CaCl₂ -Lösung (0.05 mol/L), in der zuvor 1 gew.% Pluronic F68 (auch bekannt unter Poloxamer 188) gelöst wurden, in einem Vial durch schütteln gemischt. Die CaF₂ Nanopartikel bilden sich sofort, was man an der leichten opaleszens der erstandenen Dispersion erkennen kann.

Mehr als 90% der eingesetzten Radioaktivität ist in den erfindungsgemäßen Partikeln gebunden.

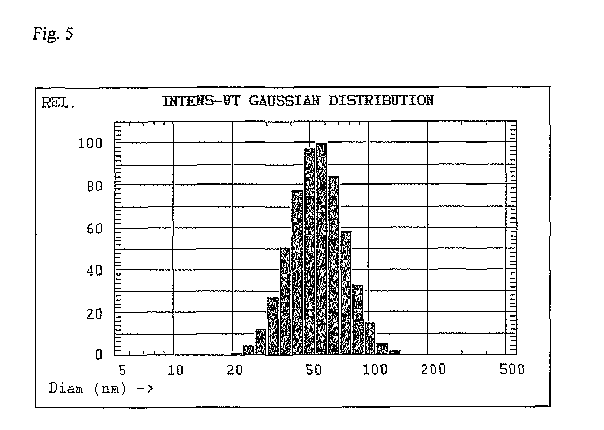

Die Bestimmung der Teilchengrößenverteilung mit Dynamischer Lichtstreuung liefert eine mittlere Partikelgröße von 64.5 nm (intensitätsgewichtet). Eine exemplarische Teilchengrößenverteilung ist in Figur 5. dargestellt.

Das Experiment wird sieben mal wiederholt und die Teilchengröße mit DLS bestimmt. Nach 24h wird erneut die Teilchengrößenverteilung gemessen. Die Ergebnisse sind in Tabelle 1 zusammengestellt.

**Tab. 1: Mittlere Partikelgröße von F68 stabilisierten CaF₂ Nanopartikel direkt nach Herstellung und 24 Stunden später.**

| Probe | Intensity-Weighted [nm] | Stabilität [24h] |
|---|---|---|
| 1 | 64,5 | 66,7 |
| 2 | 64,6 | 66,1 |
| 3 | 61,3 | 64,0 |
| 4 | 57,1 | 60,4 |
| 5 | 59,4 | 70,8 |
| 6 | 54,8 | 61,5 |
| 7 | 57,3 | nicht bestimmt |
| Mittelwert [nm] | 60,3 | 64,9 |
| STABW [nm] | 4,0 | 3,5 |
| VK[%] | 6,6 | 5,3 |

Die Versuchsreihe zeigt eine sehr gute Reproduzierbarkeit der Nanopartikelgröße (Variationskoeffizient <7%). Die Teilchengröße nach 24 Stunden Standzeit bei Raumtemperatur ist im Mittelwert von 60.3±4.0 nm (n=7) leicht auf 64.9±3.5 nm (n=6) gestiegen, dieser Anstieg ist aber noch im Rahmen der einfachen Standardabweichung der Versuchreihe und deshalb statistisch nicht signifikant. Demzufolge sind die CaF₂ Nanopartikel bezüglich der Teilchengrößenverteilung zumindest 24 h stabil.

### 1.2.3 Kontrolle der CaF₂ Nanopartikel Teilchengröße

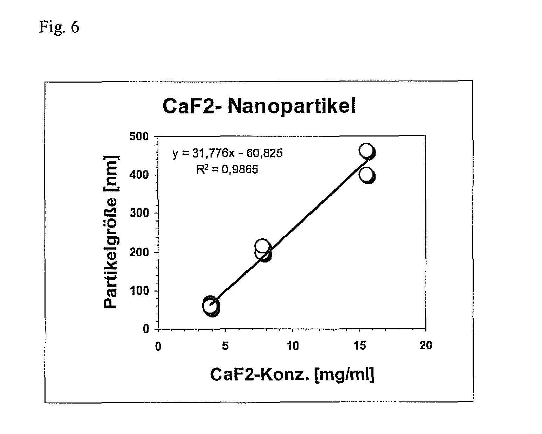

Analog zu oben beschriebener Versuchsreihe werden CaF₂ Nanopartikel aus stärker konzentrierten KF-Lösung und CaCl₂ -Lösung hergestellt. Zusammen mit den Ergebnissen zur Prüfung der Reproduzierbarkeit (Beispiel 1.2.2) sind in Figur 6 die Partikelgrößen als Ergebnis der Mischung von jeweils 1mL 0.2 mol/L KF- mit 0.1 1 mol/L CaCl₂ -Lösung bzw. 0.4 mol/L KF- mit 0.2 mol/L CaCl₂ -Lösung dargestellt. Die Partikelgröße wächst mit zunehmender Konzentration von etwa 60 nm bis auf etwa 400 nm an.

### Beispiel 2 : Nanopartikuläre schwerlösliche, anorganische [11]C bzw. [15]O Carbonatverbindung und deren Nutzung als PET Diagnostikum

### Beispiel: Nanopartikuläres Calciumcarbonat, CaCO₃, als PET-Diagnostikum

Die Nuklide werden nach folgender Target Reaktion im Cyclotron hergestellt:

| Target Material Nuklid | Target Reaktion | Produkt | |
|---|---|---|---|
| N₂+0.5% O2 ... | N14 → (p, α) → C11 ... | CO₂ | [11]C |
| N₂ + 2% CO2 ... | N14 → (d, n) **→** 015... | CO₂ | [15]O |

| | | | |
|---|---|---|---|
| C[11] t1/2=20.7min O[15] t1/2=2 min. | | | |

Fällungsreaktionsgleichung:

CO₂ + H₂O ↔ H₂CO₃

Ca(OH)₂ + H₂CO₃ → CaCO₃↓ + 2 H₂O

Gemäß der Fällungsreaktion werden in Gegenwart von geeigneten Stabilisatoren, beispielsweise F68, Nanopartikel gebildet. Calcophile-Stabilisatoren wie Blockcopolymere von Typ Polyacrylsäure-block-PEG, Phosphat-funktionalisierte-Polymere-block-PEG, Phosphonat-funktionalisierte-Polymere-block-PEG sind dafür besonders gut geeignet. Eine chemische Kopplung der Stabilisatoren zu zielspezifischen Liganden wie Antikörper, Antikörperfragmenten, Peptiden etc. vom Typ Ligand-PEG-block-calcophiles-Polymer liefert Nanopartikel, die zielgerichtet an entsprechende Targets im Patienten binden können.

### Beschreibung der Figuren :

**Fig. 1****:**
   **Figur 1** **Typ 1** beschreibt erfindungsgemäße Partikel aus der oben beschriebenen Gruppe der schwerlöslichen anorganischen Verbindungen (C), die stabilisatorfrei koagulationsgehemmt sind. Hier tragen adsorbierte Wassermoleküle oder/und die elektrische Aufladung der Partikeloberfläche zur Stabilisierung der Partikel bei (auch Sol genannte Systeme).
**Figur 1** **Typ 2** sind erfindungsgemäße Partikel, die mit grenzflächenaktiven Molekülen gegen Aggregation stabilisiert sind. Neben den gängigen i.v. applizierbaren Stabilisatoren wie beispielsweise Poloxamere, können auch speziell maßgeschneiderte Blockcopolymere eingesetzt werden. Hier sind z.B. so genannte doppelhydrophile A-Block-B-Copolymere zu nennen, deren Block A koodinierende/stabilisierende Fähigkeiten besitzt und der zweite Block B die Partikel vor Aggregation schützt und oft aus Polyethylenglycol (PEG) besteht. Darüber hinaus können die erfindungsgemäßen Partikel mit weiteren Materialen beschichtet werden, z.B. mit klassischen Beschichtungsmaterialien wie Kohlenhydraten, Polyethylenglycolen, Polyacryl- ader-methacylsäuren, Fettsäuren, Silica oder/und Silan etc..
**Figur 1** **Typ 3** zeigt eine weitere Ausführungsform der erfindungsgemäßen Partikel mit zielspezifischer Partikelstruktur, die durch zusätzliches Ankoppeln von spezifischen Liganden erhalten werden. Diese Liganden können beispielsweise Antikörper, deren Fragmente, Peptide, Aptamere, Darpine oder andere kleine Moleküle sein, die eine hohe Affinität zu krankheitsspezifischen Rezeptoren besitzen. Die Liganden können sowohl über chemische Modifizierung der zur Stabilisierung verwendeten Substanzen gekoppelt werden als auch direkt am erfindungsgemäßen Partikel verankert sein.
**Fig. 2****:** Zusätzlich kann der Kern der erfindungsgemäßen Partikel aus zwei verschiedenen Materialen bestehen (Kern-Schale-Struktur; **Figur 2** **Typ 1.1, 1.2, 1.3**). Dabei kann in einer bevorzugten Ausführungsform das innere C2 (oder äußere C1) aus einem weiteren diagnostisch nutzbaren Mittel bestehen. Beispielsweise können Magnetite als Nukleationskeime genutzt werden und die oben beschriebenen schwerlöslichen anorganischen Verbindungen auf diesen gefällt bzw. aufgebracht werden.
   In einer weiteren Ausführungsform kann die Schale der erfindungsgemäßen Partikel vom Typ 1.1, 1.2 und 1.3 aus jeder pharmazeutisch annehmbaren Hülle bestehen.
**Fig. 3****: Darstellung des Mechanismus zum passiven Targeting.** Gesunde Blutgefässe haben ein geschlossenes Endothel. Sie sind dicht und halten größere Moleküle wie z.B. Albumin oder auch Nanopartikel zurück in der Blutbahn. Das Endothel von Blutgefässen in Tumoren hat Löcher, durch diese kleine erfindungsgemäße Partikel leicht aus der Blutbahn ins Gewebe diffundieren können. Da überdies der Lymphtransport im Tumorgewebe vermindert ist, reichern sich die erfindungsgemäßen Partikel im Tumor an - allein durch die richtige nanoskalige Größe.
**Fig. 4****:** Fotographie einer applikationsfertigen CaF₂ Dispersion gemäß Beispiel 1.2.1. Die CaF₂ Nanopartikel sind an der leichten opaleszens der erstandenen Dispersion zu erkennen.
**Fig. 5****:** Exemplarische Teilchengrößenverteilung von CaF₂ Nanopartikeln gemäß Beispiel 1.2.2 bestimmt durch Dynamische Lichtstreuung.
**Fig. 6****.** Kontrolle der CaF₂ Nanopartikel Teilchengröße anhand von 11 unabhängigen Experimenten analog zur beschriebenen Herstellungsanleitung nach Beispiel 1, insbesondere gemäß Beispiel 1.2.3. Der mittlere Durchmesser der Teilchengrößenverteilung kann so von 60nm bis etwa 400nm eingestellt werden

| *Anhang:* Auszug nutzbarer Isotope (unvollständige Auswahl) s = Sekunden; m = Minuten; d = Tage; a = Jahre | | | | |
|---|---|---|---|---|
| Isotop | Halbwertszeit | Zerfallsenergie (MeV) | Spinparität | Zerfallsart(en) oder Häufigkeit (%) |
| 31S | 2,572 s | 5,396 | 1/2+ | K/β+=100 |
| 150 | 122,24 s | 2,754 | 1/2- | K/β+=100 |
| 30P | 2,498 m | 4,232 | 1+ | K/β+=100 |
| 13N | 9,965 m | 2,220 | 1/2- | K/β+=100 |
| 65Ga | 15,2 m | 3,255 | 3/2- | K/β+=100 |
| 11C | 20,39 m | 1,982 | 3/2- | K/β+=100 |
| 68Ga | 67,629 m | 2,921 | 1+ | K/β+=100 |
| 18F | 109,77 m | 1,656 | 1+ | K/β+=100 |
| 131Ba | 11,50 d | | 1/2+ | K/β+=100 |
| 26Al | 7,17-105 a | 4,004 | 5+ | K/β+=100 |
| | | | | |
| ¹²²Ba | 1,95 m | | 0+ | K/β⁺=100 |
| ¹²³Ba | 2,7 m | | 5/2+ | K/β⁺=100 |
| ¹²⁴Ba | 11,0 m | | 0+ | K/β⁺=100 |
| ¹²⁵Ba | 3,5 m | | 1/2(+) | K/β⁺=100 |
| ¹²⁶Ba | 100 m | | 0+ | K/β⁺=100 |
| ¹²⁷Ba | 12,7 m | | 1/2+ | K/β⁺=100 |
| ¹²⁹Ba | 2,23 h | | 1/2+ | K/β+=100 |
| 129mlBa | 2,16 h | | 7/2+ | K/β+<100 |
| 131Ba | 11,50 d | | 1/2+ | K/β+=100 |
| | | | | |
| ⁵¹Fe | 305 ms | 8,020 | 5/2- | K/β⁺=100 |
| ⁵²Fe | 8,275 h | 2,372 | 0+ | K/β⁺=100 |
| ^{52ml}Fe | 45,9 s | 9,192 | (12+) | K/β⁺=100 |
| ⁵³Fe | 8,51 m | 3,743 | 7/2- | K/β⁺=100 |

## Patentansprüche

1. Pharmazeutisches Mittel enthaltend eine partikuläre anorganische Matrix, die neben der natürlichen Isotopenverteilung der Strukturtypen-bildenden Elemente der Anionen bzw. Kationen Anteile an Positronen-emittierenden Nukliden enthält, wobei die Anzahl der Positronen-emittierenden Nuklide pro partikulärer anorganischer Matrix größer gleich 1 ist.

2. Pharmazeutisches Mittel gemäß Anspruch 1, wobei die partikuläre anorganische Matrix in Wasser und in physiologischen Flüssigkeiten schwerlösliche anorganische Verbindungen sind.

3. Pharmazeutisches Mittel gemäß Anspruch 1 oder 2, wobei die partikuläre anorganische Matrix ausgewählt ist aus der Gruppe umfassend Topas (Al₂F₂)[SiO₄], Chiolith Na[Al₃F₄], Wavellit Al₃(PO₄)₂(OH, F)₂, Calciumcarbonat (CaCO₃), Maghemit (γ-Fe₂O₃), Zeolithe (allg. Formel Mₙ[(AlO₂)ₓ(SiO₂)_{y}] (M= Metall z.B. Na)), Magnetit (Fe₃O₄), Bariumsulfat (BaSO₄), Galliumphosphat (GaPO₄), Apatit bzw. Fluorhydroxylapatit (Ca₅(PO₄)₃(OH, F) = 3Ca₃(PO₄)₂ * Ca(OH, F)₂) und Flußspat (CaF₂).

4. Pharmazeutisches Mittel gemäß einem der Ansprüche 1 bis 3, wobei die Positronen-emittierenden Nuklide ausgewählt sind aus der Gruppe umfassend [15]O, [30]P, [13]N, [65]Ga,[11]C, [131]Ba, [26]Al,und [68]Ga und [18]F.

5. Pharmazeutisches Mittel gemäß einem der Ansprüche 1 bis 4, wobei die partikuläre anorganische Matrix einen Durchmesser von 0,1nm bis 100µm aufweist.

6. Pharmazeutisches Mittel gemäß einem der Ansprüche 1 bis 5, wobei die partikuläre anorganische Matrix in einer pharmazeutischen Hülle ist.

7. Pharmazeutisches Mittel gemäß einem der Ansprüche 1 bis 6, wobei die partikuläre anorganische Matrix an target-spezifische Liganden angekoppelt ist.

8. Verfahren zur Herstellung eines pharmazeutischen Mittels gemäß einem der Ansprüche 1-7, umfassend mindestens einen der folgenden Schritte:
(i) Mischen mindestens zweier Lösungen löslicher Salze gemäß einer Fällungsreaktionsgleichung, wobei mindestens ein Salz mit Positronen-emittierenden Nukliden angereichert ist und wobei die Kationen des einen Salzes mit den Anionen des anderen Salzes ausfallen und wobei der Positronen-emittierende Anteil Teil der gefällten Verbindung ist und somit eine partikuläre anorganische Matrix bildet, die neben der natürlichen Isotopenverteilung der Strukturtypen-bildenden Elemente der Anionen bzw. Kationen Anteile an Positronen-emittierenden Nukliden enthält.
(ii) Bildung einer Suspension gemäß einer Fällungsreaktionsgleichung enthaltend die partikuläre anorganische Matrix, die neben der natürlichen Isotopenverteilung der Strukturtypen-bildenden Elemente der Anionen bzw. Kationen Anteile an Positronen-emittierenden Nukliden enthalten.
(iii) Mischen mindestens zweier Lösungen löslicher Salze gemäß einer Fällungsreaktionsgleichung zur Bildung einer anorganischen Matrix und nachträglicher Zugabe der Positronen-emittierenden Nuklide in anionischer bzw. kationischer Form.

9. Pharmazeutische Zusammensetzungen, wobei mindestens 0,001% der in der Zusammensetzung enthaltenden Partikel, anorganische Partikel gemäß einem der Ansprüche 1 bis 7 sind.

10. Pharmazeutisches Mittel gemäß einem der Ansprüche 1 bis 7 zur Verwendung in der diagnostischen Bildgebung.

11. Pharmazeutisches Mittel gemäß einem der Ansprüche 1 bis 7 zur locoregionalen Therapie einer Erkrankung.
